Europäisches Patentamt

(19) European Patent Office

Office européen des brevets

(11) Publication number: **0 179 786**
**B1**

(12) **EUROPEAN PATENT SPECIFICATION**

(45) Date of publication of patent specification: **16.05.90**

(21) Application number: **85901488.8**

(22) Date of filing: **21.03.85**

(86) International application number:
**PCT/GB85/00110**

(87) International publication number:
**WO 85/04419 10.10.85 Gazette 85/22**

(51) Int. Cl.⁵: **C 12 N 15/00,** C 12 P 21/00,
C 12 P 21/02

(54) **HIGH COPY NUMBER EXPRESSION VECTORS.**

(30) Priority: **22.03.84 GB 8407498**

(43) Date of publication of application:
**07.05.86 Bulletin 86/19**

(45) Publication of the grant of the patent:
**16.05.90 Bulletin 90/20**

(84) Designated Contracting States:
**AT BE CH DE FR GB IT LI LU NL SE**

(56) References cited:
**EP-A-0 104 061**
**WO-A-84/01171**

**Proceedings of the National Academy of
Sciences USA, vol. 80, June 1983 T.Som et al.:
"Regulatory regions of ColE1 that are involved in
determination of plasmid copy number", pages
3232-3236, see page 3234, first column
Nature, vol. 283, 10 January 1980 A.J. Twigg et
al.: "Trans-complementable copy-number
mutants of plasmid Col E1", pages 216-218, see
the first paragragh**

(73) Proprietor: **Biogen, Inc.
Fourteen Cambridge Center
Cambridge, Massachusetts 02142 (US)**

(72) Inventor: **PANAYOTATOS, Nikos
17 Pre-Jerome
CH-1205 Geneva (CH)**

(74) Representative: **Bannerman, David Gardner
et al
Withers & Rogers 4 Dyer's Buildings Holborn
London, EC1N 2JT (GB)**

Courier Press, Leamington Spa, England.

# EP 0 179 786 B1

(56) References cited:

Proceedings of the National Academy of Sciences USA, vol. 79, October 1982, G.Cesareni et al."Control of Co1E1 DNA replication: the rop gene product negatively affects transcription from the replication primer promoter", pages 6313-6317, see the abstra

Chemical Abstracts, vol. 97, no. 9, August 1982(Columbus, Ohio, US) A.Rosner et al.: "Screening for highly active plasmid promoters via fusion to beta-galactosidase gene", see page 139, abstract no. 67053r

Chemical Abstracts, vol. 101, no. 7, 13 August 1984 (Columbus, Ohio,US) J.E.L. Larsen et al.:"Low-copy-number plasmid cloning vectors amplifiable by derepression of an inserted foreign promoter", see page 133, abstract no. 49322z

Nucleic Acids Research, vol. 12, no. 6, 25 March 1984 N.Panayotatos: "DNA replication regulated by the priming promoter", pages 2641-2648, see the entire document

## Description

### Technical field of invention

This invention relates to improved high copy number expression vectors and to methods for increasing the copy number of such vectors and for expressing cloned genes using them. The vectors and methods disclosed in this invention are characterized by an increase in copy number of the vector in an appropriate host. As will be appreciated from the disclosure to follow, these vectors and methods may be used to improve the production of various polypeptides, proteins and amino acids in host cells transformed with DNA sequences coding for those products.

### Background art

The level of production of a protein in a host cell is governed by three major factors: the number of copies of its gene within the cell, the efficiency with which those gene copies are transcribed and the efficiency with which the resultant messenger RNA ("mRNA") is translated. The number of copies of a cloned gene within a cell depends on the number of copies of the expression vector containing that gene present in the cell during expression.

Vector copy number is controlled by the interaction of three elements in ColEl-type plasmids: the RNA which primes DNA replication ("primer RNA"), the small "RNA I" and, the protein product of the rop gene. These three elements are coded for by and act within a 1300 base pair region surrounding the origin of DNA synthesis (G. Cesareni et al., "Control of ColEl DNA Replication: The rop Gene Product Negatively Affects Transcription from the Replication Primer Promoter," Proc. Natl. Acad. Sci. USA 79, 6313—17 (1982)). The synthesis of primer RNA and RNA I is controlled by promoters $P_m$ and $P_l$, respectively. Promoters $P_m$ and $P_l$ are constitutive, i.e., they continually promote expression of genes operatively linked to them (E. M. Wong et al., "Temperature-Sensitive Copy Number Mutants of ColEl are Located in an Ultranslated Region of the Plasmid Genome", Proc. Natl. Acad. Sci. USA 79, 3570—74 (1982)).

Both the rop gene product and RNA I inhibit DNA replication, and hence copy number, by interaction with primer RNA. However, mutations in the genes coding for either RNA I or primer RNA, which interfere with the interaction between the two RNA's, have resulted in an increase in DNA replication and accordingly an increase in the copy number of the plasmid within a host cell (J. Tomizawa and T. Itoh, "Plasmid ColEl Incompatibility Determined by Interaction of RNA I with Primer Transcript", Proc. Natl. Acad. Sci. USA 78, 6096—6100 (1981)); (G. Cesareni et al., supra).

In addition to copy number, the particular characteristics of the expression control sequence used to promote gene expression to produce a desired product is also important. For example, expression control sequences may be constitutive or controllable. Preferred expression control sequences are controllable, e.g., they may be switched off to enable the host cells to propagate without excessive build-up of possibly cell-toxic products and then switched on to promote synthesis under the control of these expression control sequences of large amounts of the desired products.

Several controllable expression control sequences, which satisfy some of the criteria set forth above, have been employed to express DNA sequences coding for proteins and polypeptides in various hosts. These include, for example, the operator, promoter and ribosome binding and interaction sequences of the lactose operon of E. coli ("the lac system") (e.g., K. Itakura et al., "Expression in Escherichia coli of a Chemically Synthesized Gene for the Hormone Somatostatin", Science 198, 1056—63 (1977); D. V. Goeddel et al., "Expression in Escherichia coli of Chemically Synthesized Genes for Human Insulin", Proc. Natl. Acad. Sci. USA 76, 106—10 (1979)), the corresponding sequences of the tryptophan synthetase system of E. coli ("the trp system") (J. S. Emtage et al., "Influenza Antigenic Determinants are Expressed from Haemagglutinin Genes Cloned in Escherichia coli", Nature 283, 171—74 (1980); J. A. Martial et al., "Human Growth Hormone: Complementary DNA Cloning and Expression in Bacteria", Science 205, 602—06 (1979)) and the major operator and promoter regions of phage λ ("the $P_L$ system") (H. Bernard et al., "Construction of Plasmid Cloning Vehicles that Promote Gene Expression from the Bacteriophage Lambda $P_L$ Promoter", Gene 5, 59—76 (1979); E. Remaut et al., Gene, 15, pp. 81—93 (1981)), and combinations of these sequences.

The lac system is controlled by a repressor protein, the product of regulator gene i. It is activated by the addition of an inducer—lactose or isopropyl-thiogalactoside ("IPTG"), a lactose analog. The trp system is also controlled by the level of tryptophan or derivatives thereof. The $P_L$ system on the other hand is temperature controlled. It is induced by increasing the temperature of the culture from 28°C to 42°C.

In view of the diverse products now being made by recombinant DNA technology, methods to increase the copy number of genes coding for those products and hence their level of production would be advantageous. Preferably, these methods should also be combined with controllable expression control sequences.

### Disclosure of the invention

This invention relates to improved high copy number expression vectors and methods for increasing the copy number of such vectors and for expressing cloned genes using them. We have discovered that when the interaction between the elements controlling DNA replication (primer RNA, RNA I and the rop gene product) is disturbed, priming of DNA synthesis becomes rate-limiting. By inactivating or deleting the promoter of primer RNA ($P_m$) and using another promoter, and more preferably a controllable promoter, to promote primer RNA synthesis, we have found that we can regulate the rate of DNA replication, and hence plasmid copy number.

Accordingly, this invention provides expression vectors, wherein the promoter of primer RNA ($P_m$) has been inactivated or deleted and another promoter has been operatively linked to the coding sequence of primer RNA.

Preferably, the promoter that is used instead of the deleted or inactivated promoter of primer RNA in the expression vectors of this invention is characterized by at least one promoter selected from the group consisting of the promoter of RNA I, the promoter of the *lac*UV5 gene and derivatives thereof.

Most preferably, the expression vectors of this invention are also characterized by a mutation or deletion which inactivates the *rop* gene product. In the presence of an inactivated *rop* gene product, the use of the promoter of RNA I or of the *lac*UV5 gene to promote the synthesis of primer RNA produces a vector with a copy number 40 times greater than a vector containing a wild-type $P_m$ and a wild-type *rop* gene.

The vectors of this invention also include at least one restriction site wherein a DNA sequence encoding a desired protein or polypeptide may be inserted into said vector and operatively linked there to an expression control sequence. The latter expression control sequence may include the promoter that now controls the synthesis of primer RNA or it may be an expression control sequence present elsewhere in the vector. More preferably, the expression control sequence used to regulate the expression of the desired product is controllable, not constitutive.

More particularly, this invention relates to the improved expression vectors, to the improved vectors and to the methods as defined in the claims appended herein.

As will be appreciated from the description of this invention, the expression vectors and methods of this invention permit the high level expression of prokaryotic and eukaryotic products encoded for by DNA sequences operatively linked to expression control sequences in the vectors of this invention.

Brief description of the drawings

Figures 1, 2 and 3 are schematic outlines of one embodiment of making and using an expression vector of this invention.

Figure 4 is a schematic outline of another embodiment of making and using an expression vector of this invention.

Figure 5 is a schematic outline of another embodiment of making and using an expression vector of this invention.

Figure 6 is a schematic outline of another embodiment of making and using an expression vector of this invention.

Figure 7 is a schematic depiction of pLA512, one of the expression vectors of this invention.

Figure 8 is a schematic depiction of pLA51, another of the expression vectors of this invention.

Figure 9 is an autoradiograph which demonstrates DNA replication of plasmid pLA51 in *E. coli* W3110($i^qL8$) at increasing concentrations of IPTG.

Best mode of carrying out the invention

In order that the invention herein described may be more fully understood, the following detailed description is set forth.

In the description the following terms are employed:

Nucleotide

A monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is called a nucleoside. The base characterizes the nucleotide. The four DNA bases are adenine ("A"), guanine ("G"), cytosine ("C"), and thymine ("T"). The four RNA bases are A, G, C and uracil ("U").

DNA sequence

A linear array of nucleotides connected one to the other by phosphodiester bonds between the 3' and 5' carbons of adjacent pentoses.

Codon

A DNA sequence of three nucleotides (a triplet) which encodes, through its template or messenger RNA ("mRNA"), an amino acid, a translation start signal or a translation termination signal. For example, the nucleotide triplets TTA, TTG, CTT, CTC, CTA and CTG encode the amino acid leucine ("Leu"), TAG, TAA and TGA are translation stop signals and ATG is a translation start signal.

Polypeptide

A linear array of amino acids connected one to the other by peptide bonds between the α amino and carboxy groups of adjacent amino acids.

Gene

A DNA sequence which encodes through its mRNA a sequence of amino acids characteristic of a specific polypeptide.

Transcription

The process of producing mRNA from a gene or DNA sequence.

Translation

The process of producing a polypeptide from mRNA.

Expression

The process undergone by a DNA sequence or gene to produce a polypeptide. It is a combination of transcription and translation.

Plasmid

A nonchromosomal, double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance (Tet$^R$) transforms a cell previously sensitive to tetracycline into one which is resistant to it. A host cell transformed by a plasmid or vector is called a "transformant".

Phage or bacteriophage
Bacterial virus many of which consist of DNA sequences encapsidated in a protein envelope or coat ("capsid").

Cloning vehicle or vector
A plasmid, phage DNA or other DNA sequence which is able to replicate in a host cell, characterized by one or a small number of endonuclease recognition or restriction sites at which such DNA sequences may be cut in a determinable fashion without attendant loss of an essential biological function of the DNA, e.g., replication, production of coat proteins or loss of promoter or binding sites, and which contains a marker suitable for use in the identification of transformed cells, e.g., tetracycline resistance or ampicillin resistance.

Cloning
The process of obtaining a population of organisms or DNA sequences derived from one such organism or sequence by asexual reproduction.

Recombinant DNA molecule or hybrid DNA
A molecule consisting of segments of DNA from different genomes (the entire DNA of a cell or virus) which have been joined end-to-end and have the capacity to infect some host cell and to be maintained therein.

Expression control sequence
A sequence of nucleotides that controls and regulates expression of genes when operatively linked to those genes. It includes the promoter, ribosome binding sites and other sequences useful in the expression of genes that are operatively linked to those expression control sequences. The term "operatively linked" includes having an appropriate start signal in front of the gene encoding the desired product and maintaining the correct reading frame to permit expression of the inserted gene under the control of the expression control sequence and synthesis of the desired product encoded for by that gene.

The host cells of this invention
Any of a large number of available and well known host cells may be used in the host-expression vector combinations of this invention. The selection of a particular host is dependent upon a number of factors recognized by the art. These include, for example, compatibility with the chosen expression vector, toxicity of the proteins encoded for by the hybrid plasmid, ease of recovery of the desired protein, expression characteristics, biosafety and costs. A balance of these factors must be struck with the understanding that not all hosts may be equally effective for the expression of a particular DNA sequence in the expression vectors and methods of this invention.

Within these general guidelines, useful hosts may include strains of E. coli, Pseudomonas, Bacillus, Streptomyces, yeast and other fungi, animal, insect or plants hosts, such as animal (including human) or plant cells in culture or other hosts known the art.

The preferred host cells of this invention are E. coli strains MC1061, HB101 and W3110(i$^q$).

Promoters used instead of P$_m$ to promote primer RNA in the vectors of this invention
The promoters used instead of the inactivated or deleted promoter of primer RNA in the expression vectors of this invention may consist of either controllable or constitutive promoters. These include promoters from the E. coli lac system, the E. coli trp system, the TAC system, the TRC system, the major operator and promoter regions of phage λ, the control region of fd coat protein and other sequences known to control the expression of genes of prokaryotic or eukaryotic cells and their viruses or combinations thereof. These promoters are preferably characterized by at least one promoter selected from the group consisting of the promoter of RNA I, the promoter of the lacUV5 gene and derivatives thereof. These promoters are hereinafter designated P$_I$, and P$_{LAC5}$, respectively. Most preferably, the promoter is controllable. Accordingly, while P$_{LAC5}$ is preferred, other controllable promoters may also be used in the vectors of this invention to promote the synthesis of primer RNA. It should, however, be understood that not all promoters may function with equal efficiency in the vectors of this invention.

Although in one illustrative embodiment of this invention, the P$_I$ promoter (the promoter of RNA I) used instead of P$_m$ to promote the primer RNA is derived from ColEI DNA, it may also be derived from plasmids selected from the group consisting of pBR322, R$_1$, cloDF13 and other similarly organized replicons and their derivatives.

Expression of DNA sequences in the vectors of this invention
The vectors of this invention may be employed to control the expression of DNA sequences coding for a desired eukaryotic, prokaryotic or viral polypeptide in a variety of ways.

In one preferred embodiment of this invention, we insert a DNA sequence encoding the desired polypeptide at a restriction site downstream from and operatively linked to an expression control sequence that includes the promoter used instead of the promoter of primer RNA (P$_m$), but upstream from the origin of DNA replication.

In the embodiment of this invention where the promoter used instead of Pm to promote primer RNA is also used to express a desired protein or polypeptide, operators, ribosome binding and interaction sequences, such as the Shine-Dalgarno sequences and other DNA sequences related to the regulation of expression of genes must also be employed together with the promoter to form an expression control sequence useful to control the expression of DNA sequences operatively linked to it. Such additional sequences, for example, include sequences from MS2, mu, bacteriophage T7, phage λ, and other like systems. Most preferably, sequences from bacteriophage T7 are employed with these promoters in the expression vectors of this invention.

In this embodiment of the invention, transcription begins at the expression control sequence, reads through the DNA sequence encoding the selected polypeptide and primes the origin of DNA replication. In this manner, the same expression control sequence simultaneously controls transcription of the DNA sequence encoding the desired polypeptide and the priming of the origin of DNA replication.

In another, more preferred embodiment, of this invention, we insert the DNA sequence encoding the desired polypeptide outside of the origin of DNA replication and operatively link it there to an expression control sequence.

Expression control sequences useful in this embodiment of the invention are well known in the art. They include both controllable and consitutive sequences. E.g., the *E. coli lac* system, the *E. coli trp* system, the TAC system, the TRC system, the major operator and promoter regions of phage λ, the control regions of fd coat protein and other sequences known to control the expression of genes of prokaryotic and eukaryotic cells and their viruses or combinations thereof. Preferably, controllable expression control sequences are used. Such expression control sequences, of course, also include the necessary operators, ribosome binding sites and interactive sequences as described above.

Most preferably, the promoter of the expression control sequence used to express the desired gene is different from the promoter used to promote primer RNA and to initiate DNA replication in the vectors of this invention. This enables separate control of the production of a desired polypeptide and of the vector copy number. However, the two promoters may also be the same.

Restriction sites used to insert the desired DNA sequences into the expression vectors of this invention are well known. They include, for example, *Ara*I, *Pst*I, *Sal*I, *Eco*RI, *Bam*HI, *Hind*III and *Sau*3a. Methods for cleaving the vectors of this invention at that restriction site and inserting into that site a DNA sequence and operatively linking it there to the expression control sequence are also wellknown. For example, the DNA sequence encoding the desired product may be combined with an ATG, a Shine-Dalgarno sequence and a ribosome binding site and the combination inserted into a restriction site downstream of the chosen promoter so as to operatively link the DNA sequence to the expression control sequence. Alternatively, the required sequences may be inserted into the vector in a variety of separate steps.

Methods for using the expression vectors of this invention

The expression vectors of this invention are useful in expressing eukaryotic, prokaryotic and viral DNA sequences. These include DNA sequences that encode animal and human hormones, such as any of the various IFN-α's, particularly α2, α5, α7, α8, IFN-β, IFN-γ, human insulin and growth hormone, bovine growth hormone, swine growth hormone and erythropoietin, human blood factors and tissue plasminogen activator, viral or bacterial antigens, such as the core or surface antigen of HBV or the antigens of FMDV, and other useful polypeptides of prokaryotic, eukaryotic or viral origin.

Methods for expressing these DNA sequences and producing the polypeptides coded for by these sequences include transforming an appropriate host with an expression vector of this invention characterized by having the desired DNA sequence operatively linked to an expression control sequence in the vector, culturing the host under appropriate conditions of growth and expression and collecting the desired polypeptide from the culture.

An additional and important use of the expression vectors of this invention is to screen DNA libraries for promoters useful in expressing DNA sequences encoding desired polypeptides. Because the promoter of primer RNA has been inactivated or deleted in our vectors, DNA replication will not occur and the vector will not replicate unless an active promoter is used instead of the inactive or deleted promoter of primer RNA ($P_m$). Accordingly, DNA fragments from any DNA library, fragmented by restriction endonuclease treatment or other methods known in the art, may be cloned into restriction sites that are operatively linked to the sequence encoding primer RNA. The resulting clones are then used to transform an appropriate host and cultured to select those clones that have replicating plasmids. The selected promoter is then removed from the vector that characterizes the selected clones by known means and used as desired to express other DNA sequences in a variety of expression vectors.

Methods and materials

All restriction enzymes, polynucleotide kinase and $T_4$ DNA ligase were purchased from New England Biolabs. Conditions for these enzymatic reactions have been described by N. Panayotatos and R. D. Wells *J. Biol. Chem.,* 254 pp. 5555—61 (1979) and N. Panayotatos and R. D. Wells, *J. Mol. Biol.,* 135, pp. 91—109 (1979). DNA was prepared for subsequent reactions by ether extraction, followed by EtOH precipitation. Agarose and polyacrylamide gel electrophoreses were performed as described in Panayotatos and Wells,

*supra.* "Fill-in" reactions with polymerase I-large fragment (Boehringer) ("Klenow") were carried out in 20 mM Tris-HCl (pH 7.6), 10 mM MgCl$_2$, 0.5 mM EDTA, 0.25 mM dithiothreitol and 60 μM each of the four deoxynucleoside triphosphates (Sigma) for 30 min at 37°C.

Example 1

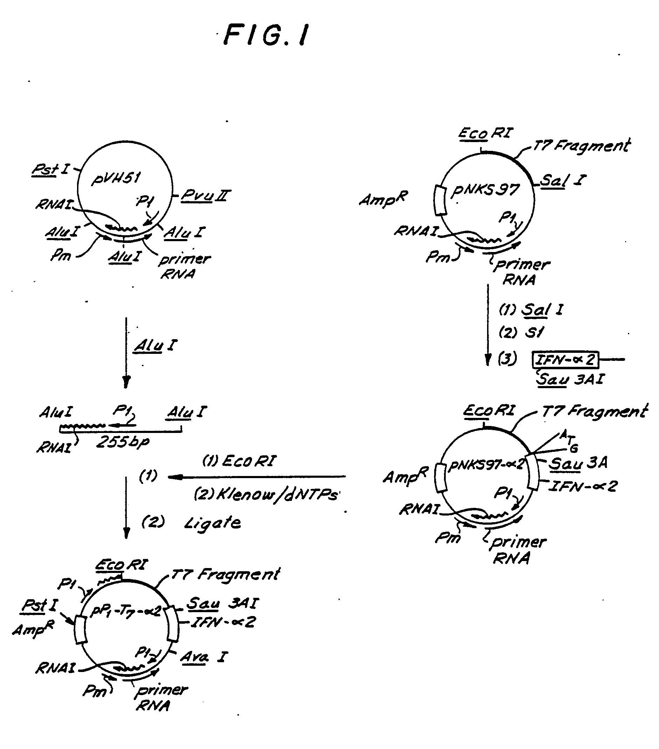

In the embodiment of this invention depicted in Figures 1, 2, and 3, we have prepared an expression vector, pRN11, that has the promoter of primer RNA (Pm) and most of the *rop* gene deleted and replaced by the promoter of RNA I (P$_I$). pRN11 also includes a T7 ribosome binding site and the DNA sequence coding for IFN-α2 operatively linked to the P$_I$ promoter.

Referring to Figure 1, we first restricted pVH51, a derivative of ColEI [V. Hershfield et al., "Characterization of a Mini-ColEI Plasmid", *J. Bacteriol.,* 126, pp. 447—53 (1976); A. Oka, "Nucleotide Sequence of Small ColEI Derivatives: Structure of the Regions Essential for Autonomous Replication and Colicin EI Immunity", *Molec. Gen. Genet.,* 172, pp. 151—59 (1979)] with *Alu*I and isolated the blunt-ended 255 base pair fragment carrying the P$_I$ promoter and part of the DNA sequence encoding RNA I by electrophoresis on a polyacrylamide gel (5%) (Figure 1).

We then restricted pNKS97 [N. Panayotatos and K. Truong, "Specific Deletion of DNA Sequences Between Preselected Bases", *Nucleic Acids Research,* 9, pp. 5679—88 (1981)] with *Sal*I, removed the overhanging ends with S1 nuclease and ligated the resulting fragment to a DNA fragment carrying the DNA sequence encoding IFN-α2 (Figure 1). This construction does not regenerate the *Sal*I site. Instead, it results in a construction having an ATG start codon (from the bacteriophage T7 fragment) attached to the TGT codon encoding the first amino acid of IFN-α2 (Figure 1). The construction is also characterized by a *Sau*3A1 restriction site following the TGT codon of the first amino acid of IFN-α2. We designated this construction pNKS97-α2. Other DNA sequences encoding desired products may be inserted into the *Sal*I site of pNKS97 in a like manner or using other methods well known in the art and employed as follows in the vectors and methods of this invention.

We next took pNKS97-α2 and restricted it with *Eco*RI, filled in the *Eco*RI residues with Klenow and dNTPs in a conventional manner and ligated to that filled-in site the blunt-ended *Alu*I fragment, described above, in a conventional manner, thereby regenerating the *Eco*RI site (Figure 1).

This ligation produced a recombinant DNA molecule comprising an expression control sequence characterized by a P$_I$ promoter and a 112 bp fragment taken from the 14.7 to 15.0% region, of bacteriophage T7 [Panayotatos and Truong, *supra*]; a DNA sequence within the replicon of the vector encoding RNA I and the primer RNA for initiation of DNA replication and their regulatory regions; and a DNA sequence encoding IFN-α2 operatively linked to the P$_I$-T$_7$ express-

ion control sequence of the vector (Figure 1). We designated this recombinant DNA molecule pP$_I$-T$_7$-α2.

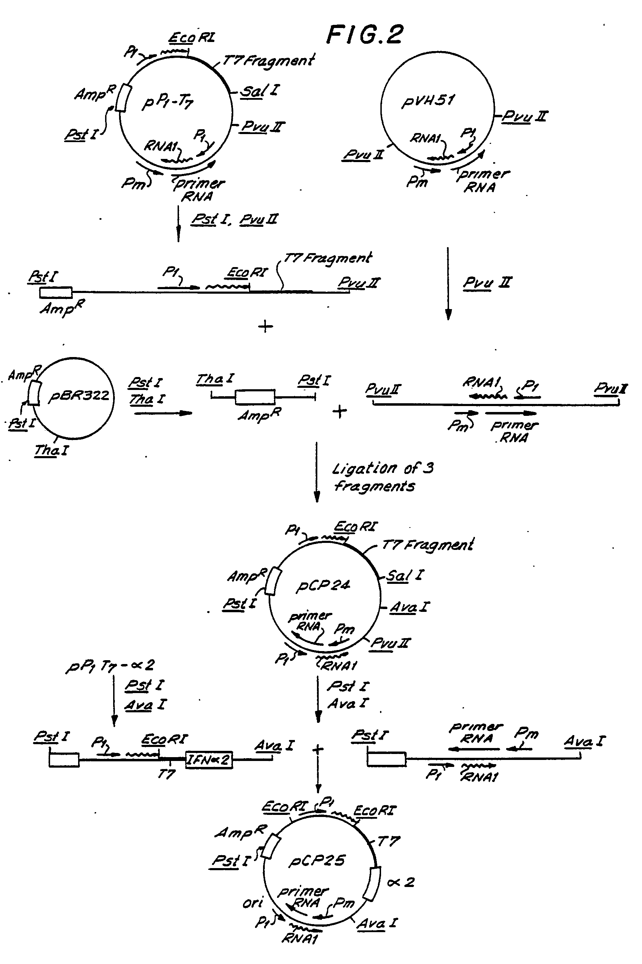

We have also prepared vector pP$_I$-T$_7$ by isolating the 255 base pair *Alu*I fragment of pVH51, as before, und ligating it to a fragment prepared by *Eco*RI restriction of pNKS97 and fill-in of the overhanging ends with Klenow/dNTPs. This vector, shown in Figure 2, is characterized by an expression control sequence characterized by promoter P$_I$ and a DNA sequence from bacteriophage T$_7$; a DNA sequence within the replicon of the vector encoding RNA I and the primer RNA for initiation of DNA replication and their regulatory regions; and a *Sal*I restriction site that permits DNA sequences encoding desired polypeptides to be inserted into the vector directly after the T7 region of the vector and an ATG start codon in that sequence so as to be operatively linked to promoter P$_I$.

Referring now to Figure 2, we restricted pP$_I$-T$_7$ with *Pst*I and *Pvu*II and collected the 2540 base pair fragment containing part of the β-lactamase gene, the P$_I$ promoter and the T$_7$ ribosome binding site. We then isolated a 509 base pair partial *Tha*I-*Pst*I fragment from the plasmid pBR322 (coordinates 3106-3611), which contains the rest of the B-lactamase gene, and a 1974 base pair *Pvu*II fragment from pVH51 containing the origin of DNA replication and the DNA sequences coding for RNA I and primer RNA. We ligated these three fragments together to produce a 5023 base pair plasmid, pCP24.

We next isolated the IFN-α2 region of pP$_I$-T$_7$-α2 (Figure 1) by treatment with *Pst*I and *Ava*I. We restricted pCP24 with *Pst*I and *Ava*I and ligated the large fragment with the α2 fragment isolated from pP$_I$-T$_7$-α2. The resulting plasmid, pCP25, catalyzed synthesis of 10—20×10$^6$ units per liter at O.D.$_{590}$ of IFN-α2 in *E. coli* HB101. The copy number of pCP25 was about 40 copies per cell.

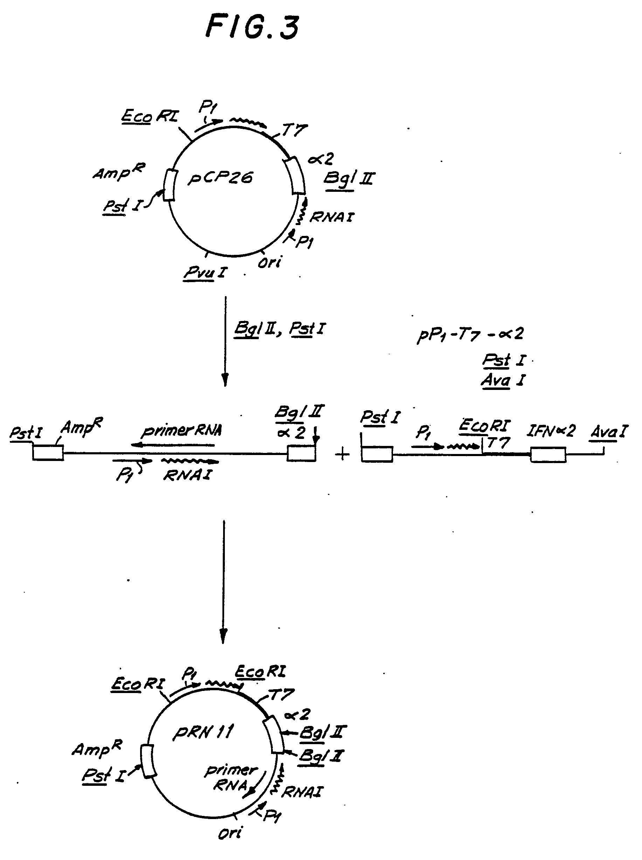

We next screened for high copy number mutants of pCP25 in *E. coli* MO by plating on 20 mg/ml ampicillin-L broth-agar. We replated four fast-growing colonies and isolated their plasmids. All four plasmids contained an extensive deletion of approximately half of pCP25, including most of the α2 gene, the downstream pBR322 sequence and some of the ColEI sequence, including the promoter for primer RNA. We designated this plasmid pCP26 (Figure 3). We determined, by restriction mapping and DNA sequencing, that the deletion was most likely caused by an homologous recombination event between two identical twelve base pair repeats present at position 216 of the α2 sequence and within the ColEI P$_I$ promoter-RNA I region. pCP26 had a copy number and level of production of β-lactamase five fold higher than did pCP25.

We next restricted pCP26 with *Bgl*II and *Pst*I and isolated a 1536 base pair fragment containing the ColEI replicon. We isolated a 2617 base pair fragment, containing the α2 gene downstream from the P$_I$ promoter, from plasmid pP$_I$-T$_7$-α2 (Figure 1) by treatment with *Pst*I and *Ava*I and

ligated it to the 1536 base pair *Bg*/II-*Pst*I fragment to form the plasmid pRN11. As a result of this sequence of steps, pRN11 lacks the promoter for primer RNA and most of the *rop* gene. The pBR322-derived DNA sequence upstream of the *Ava*I site was also deleted in this sequence of steps. The plasmid also contains an additional P$_I$ promoter and a 112 base pair fragment comprising the T7 ribosome binding site operatively linked to the DNA sequence coding for IFN-α2. In pRN11 this additional P$_I$ promoter controls the production of primer RNA and therefore the initiation of DNA replication by read-through from the α2 interferon gene to the sequences encoding the primer RNA. *E. coli* HB101 transformed with pRN11 produced IFN-α2 at levels of 5% to 10% of total cellular protein.

Example 2

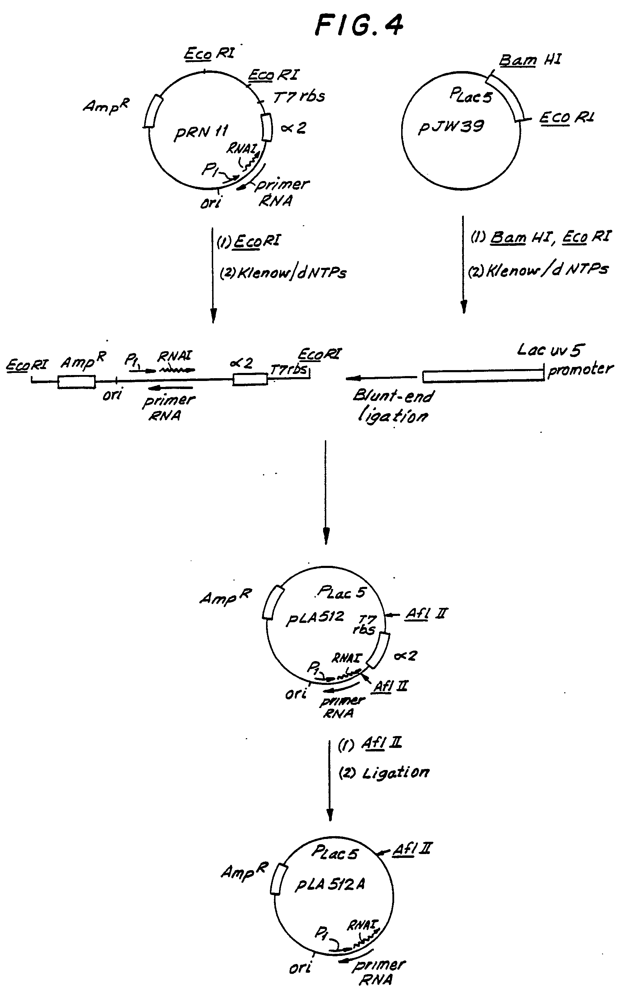

Referring now to Figure 4, in order to replace the P$_I$ promoter of pRN11 with the *lac*UV5 promoter (P$_{LAC5}$), we restricted pRN11 with *Eco*RI to remove the P$_I$ promoter and isolated the large fragment. We then filled in the *Eco*RI residues with Klenow and dNTPs in a conventional manner.

We next restricted pJW39 (T. Kovacic, personal communication) with *Bam*HI and *Eco*RI and isolated the 99 base pair fragment containing the *lac*UV5 promoter (P$_{LAC5}$). We filled in the ends with Klenow and dNTPs as above and blunt-end ligated that filled-in fragment to the large *Eco*RI fragment of pRN11 produced above.

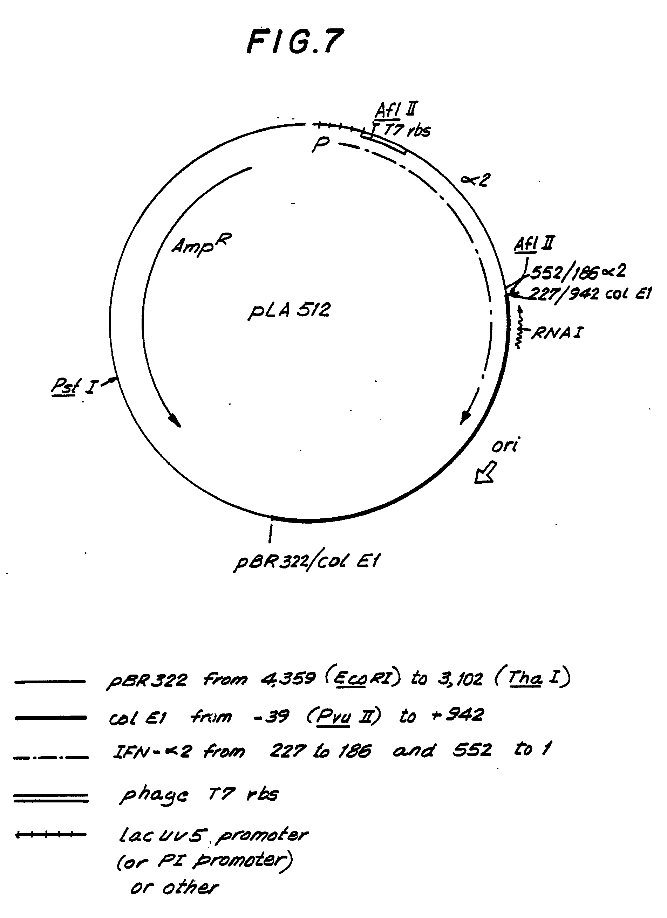

This ligation produced a reombinant DNA molecule comprising an expression control sequence characterized by the P$_{LAC5}$ promoter and the T7 ribosome binding site operatively linked to the DNA sequence encoding IFN α2 and also controlling the priming of the origin of DNA replication. We designated this recombinant DNA molecule pLA512 (see also Figure 7).

We transformed *E. coli* MC10161 with pLA512 using conventional conditions and cultured the transformed host at 37°C in L-broth, 40 µg/ml ampicillin. The P$_{LAC5}$ promoter in this expression vector not only controls DNA replication and increases the copy number of pLA512 to approximately 300 copies per cell but it also controls expression of IFN-α2. In this host-vector system we observed production of IFN-α2 at a level of about 10% of total cellular protein on appropriate addition of IPTG.

pLA512 is also useful to clone and express DNA sequences of other desired polypeptides. First, we removed the T7 ribosome binding site and the entire α2 gene by restriction of pLA512 with *Afl*II [not yet commercially available] which cuts at the 5′ end of the T7 ribosome binding site sequences and at the 3′ end of the α2 gene (Figure 4). Upon re-ligation, the *Afl*II site is restored and a plasmid is produced, having a unique *Afl*II site between the P$_{LAC5}$ promoter and the origin of DNA replication.

This *Afl*II restriction site is then used as an insertion site for other DNA sequences that code for a desired polypeptide. For example, using well-known procedures, we can insert a DNA sequence coding for the desired polypeptide operatively linked to sequences coding for a ribosome binding site and other necessary sequences into the *Afl*II site of that plasmid. As a result, the DNA sequence coding for the desired polypeptide is operatively linked to P$_{LAC5}$. On induction, as before, such a vector will provide a high copy number and a high level of expression of the DNA sequence coding for the desired product. Alternatively, another restriction site located outside of the origin may be used to insert an expression control sequence-DNA combination. The resulting vector then has the DNA sequence encoding the desired product under the control of a separate, and perhaps different, promoter than the promoter controlling the primer RNA.

Example 3

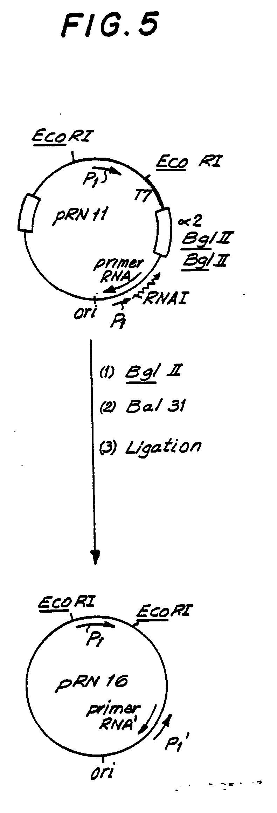

Referring now to Figure 5, we have depicted therein another embodiment of this invention wherein a vector lacking the P$_m$ promoter for primer RNA is used to select other promoters useful for expressing DNA sequences coding for desired products.

We restricted pRN11 with *Bg*/II. We next treated the large *Bg*/II fragment with Bal31 nuclease and ligated the resultant fragments under standard conditions (N. Panayotatos & K. Truong, *supra*). This treatment resulted in removal of 625 base pairs, including all of the α2 gene, part of the T7 ribosome binding site and 180 base pairs of ColEI DNA containing the RNA I coding sequence and most of the promoter controlling the production of RNA I.* We designated this recombinant DNA molecule pRN16.

We can then restrict pRN16 with *Eco*RI to remove the P$_I$ promoter and isolate the large fragment containing the origin of DNA replication. We can digest *E. coli* chromosomal DNA with *Eco*RI and combine the large pRN16 fragment with the digested DNA under standard conditions for ligation. We can then transform *E. coli* MC1061 with the mixture and culture the bacteria on plates in the presence of ampicillin to select for Amp$^R$ colonies which contain replicating plasmids, indicating the presence of a functional promoter. The promoter can then be excised from the plasmid and used in conventional ways.

Example 4

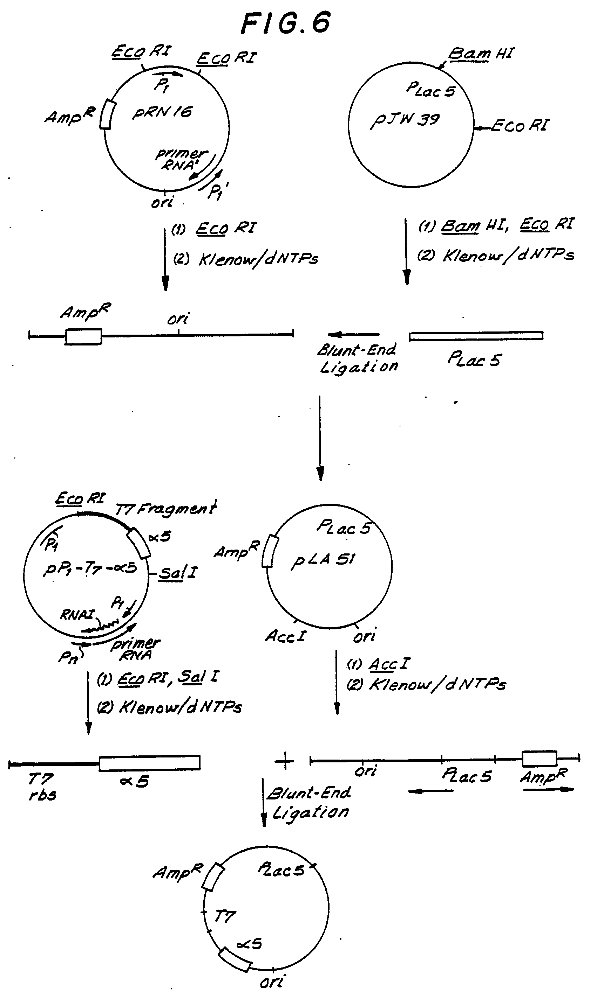

Referring now to Figure 6, we have depicted therein another embodiment of a method for producing and using an expression vector of this invention.

_____

* The P$_I$, which controlled transcription of α2 and primed the origin of DNA replication in pRN11, was not affected by this digestion. Neither was the ability of the vector to replicate in a host transformed with it impaired.

Because it may be preferable to clone a gene under the control of a promoter other than the promoter which controls the priming of the origin of DNA replication, we constructed a plasmid in which the gene coding for IFN-α5 was placed under the control of the promoter for the gene coding for ampicillin resistance.

We restricted pRN16 with *Eco*RI to remove the $P_1$ promoter and isolated the large fragment. We filled in the *Eco*RI residues with Klenow and dNTPs in a conventional manner. We then restricted pJW39 (Example 1, *supra*) with *Bam*HI and *Eco*RI and isolated the 99 base pair fragment containing the *lac*UV5 promoter, $P_{LAC5}$. We filled in the ends with Klenow and dNTPs as above and blunt-end ligated that filled-in fragment to the large *Eco*RI fragment of pRN16.

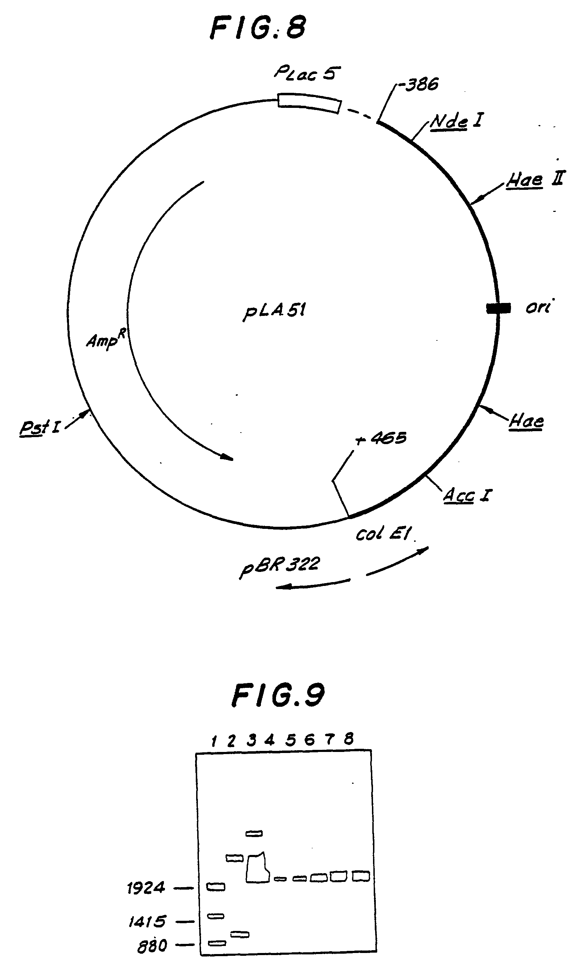

This ligation produced a recombinant DNA molecule comprising an expression control sequence characterized by the $P_{LAC5}$ promoter controlling the priming of the origin of DNA replication and a unique *Acc*I restriction site downstream from the origin of DNA replication. We designated this recombinant DNA molecule pLA51 (see also Figure 8).

We used pLA51 to clone DNA sequences encoding α5 interferon.

We first prepared $pP_1$-$T_7$-α5 using $pP_1$-$T_7$-α2. This plasmid could also have been prepared in substantially the same manner as $pP_1$-$T_7$-α2 (Figure 1).

We next restricted pLA51 with *Acc*I and filled in the *Acc*I residues with klenow and dNTPs in a conventional manner. We then restricted plasmid $pP_1$-$T_7$-α5 with *Eco*RI and *Sal*I and isolated the small fragment containing the $T_7$ ribosome binding site and the α5 gene. We filled in the staggered ends with Klenow and dNTPs as above and blunt-end ligated that filled-in fragment to the *Acc*I-linearized pLA51.

This ligation produced a recombinant DNA molecule comprising an expression control sequence characterized by the $P_{LAC5}$ promoter controlling the priming of the origin of DNA replication and the DNA sequence encoding α5 interferon operatively linked to the promoter of the gene coding for ampicillin resistance. The α5 gene is transcribed by read-through transcription from the promoter of the ampicillin resistance gene.

We transformed *E. coli* MC1061 with this plasmid using conventional conditions and cultured the transformed host at 37°C in L-broth, 40 µg/ml ampicillin. Expression of α5, however, was only to 0.2% of total cellular protein, presumably because the majority of transcripts beginning at the Amp$^R$ promoter are terminated at the end of the Amp$^R$ gene with only a small amount of read-through to the α5 gene.

In order to avoid the problem of termination before transcription of DNA sequences coding for the desired protein or polypeptide, we, using conventional techniques, can delete the terminators or introduce at the *Acc*I site a promoter, including a DNA sequence coding for a ribosome binding site, operatively linked to the DNA sequence coding for α5 or another desired protein or polypeptide.

## Example 5

pLA51 contains the $P_{LAC5}$ promoter which controls priming of the origin of DNA synthesis. Since $P_{LAC5}$ is a controllable promoter, induced by IPTG, and since in pLA51 plasmid copy number is controlled by the priming event, induction of $P_{LAC5}$ causes an increase in the copy number of pLA51.

We cultured *E. coli* W3110(i$^q$L8)* transformed with pLA51 at 37°C in 5 ml L-broth, 0.2 mM IPTG, 40 µg/ml ampicillin to early exponential phase. We then used 0.1 ml of this culture to inoculate 10 ml L-broth containing increasing concentrations of IPTG. We harvested the cells after ten generations of growth in the absence of ampicillin and purified DNA by a quick lysis procedure (R. D. Klein et al., *Plasmid 3*, 88—91 (1980)). We then restricted the plasmids with *Pvu*I and *Sal*I and loaded identical aliquots of the linearized plasmids on a 1% agarose horizontal electrophoresis gel and processed using known methods (N. Panayotatos et al., *J. Cellular Biochem. Supplement 7B* Abstract No. 765 (1983).

Referring now to Figure 9, lanes 4—8 represent aliquots of culture incubated in the presence of 0.0, 0.02, 0.06, 0.20 and 0.60 mM IPTG respectively; lane 1 contains size-marker DNA; lane 2 contains a plasmid with the same copy number as pBR322 prepared in the same manner and digested with *Pvu*I and *Sal*I and lane 3 contains pLA51 prepared after culturing in parallel in *E. coli* strain MC1061 (a strain lacking the *lac* repressor) and loaded onto the gel after digestion with *Pvu*I. Figure 9 demonstrates that pLA51 copy number increased proportionately with increasing IPTG concentration.

We have done similar experiments using pLA512 and increasing concentrations of IPTG or lactose. We observed an increase in copy number of 112-fold over the lactose and IPTG concentration ranges employed and an increase in IFN-α2 production of 1000-fold over the lactose and 120-fold over the IPTG concentration ranges employed. The final level of IFN-α2 in the presence of lactose was about 10% of total cellular protein.

Microorganism and expression vectors of this invention are exemplified by cultures deposited in the Deutsche Sammlung von Mikroorganis-

---

* *E. coli* W3110(i$^q$L8) produces ten times more *lac* repressor (B. Muller-Hill et al., "Mutants That Make More LAC Repressor", *Proc. Natl. Acad. Sci. USA 59*, 1259—63 (1968)) and fifteen times less *lac* enzymes (J. G. Scaife & J. R. Beckwith, "Mutational Alteration of the Maximal Level of Lac Operon Expression", *Cold Spring Harbor Symp. Quant. Biol. 31*, 403—09 (1967)) than a wild-type strain.

men, Goettingen, West Germany, on March 22, 1984 and identified as NP-1 to NP-4:

NP-1: *E. coli* MC 1061 (pCP26)
NP-2: *E. coli* MC 1061 (pRN11)
NP-3: *E. coli* MC 1061 (pRN16)
NP-4: *E. coli* MC 1061 (pLA51).

These cultures have been assigned accession number DSM 2927, 2928, 2929 and 2930, respectively.

## Claims

1. An improved expression vector comprising an inactive or deleted promoter of primer RNA, another promoter operatively linked to the DNA sequence encoding primer RNA in the vector, and at least one restriction site wherein a DNA sequence encoding a desired polypeptide may be inserted into such vector and operatively linked therein to an expression control sequence.

2. The expression vector according to claim 1, characterized in that said other promoter is selected from the group consisting of the promoter of RNA I, the promoter of *lac*UV5 and derivatives thereof.

3. The expression vector according to claim 1 or 2, characterized by a deletion, insertion or mutation which inactivates the *rop* gene product.

4. The expression vector according to any of claims 1 to 3, characterized in that said expression control sequence is characterized by the promoter that is operatively linked to the DNA sequence encoding primer RNA.

5. The expression vector according to claim 4, characterized in that said expression control sequence also includes sequences coding for the ribosome binding sites, including the Shine Dalgarno sequences.

6. The expression vector according to claims 1 to 5, characterized in that it is selected from the group consisting of pRN16 (accession number DSM 2929), pLA51 (accession number DSM 2930), and pLA512 (as described in the description).

7. The expression vector according to any one of claims 1 to 3, characterized in that said expression control sequence is characterized by a promoter that is not operatively linked to the DNA sequence encoding primer RNA.

8. The expression vector according to any one of claims 1 to 7, characterized in that it also includes a DNA sequence encoding a eukaryotic or prokaryotic polypeptide inserted into said vector at said restriction site and operatively linked therein to said expression control sequence.

9. The expression vector according to claim 8, characterized in that said DNA sequence is selected from the group consisting of DNA sequences encoding animal and human hormones, viral and bacterial antigens, and other eukaryotic and prokaryotic polypeptides.

10. The expression vector according to claim 9, characterized in that said DNA sequence encodes polypeptides selected from the group consisting of human and animal interferons, human and animal growth hormones, antigens of FMDV, antigens of HBV human insulin, human blood factors, tissue plasminogen activator, and erythropoietin.

11. A method for producing a polypeptide characterized by the steps of culturing a host transformed with a vector of any of claims 8 to 10 and collecting said polypeptides.

12. An improved vector for use in selecting promoters from DNA populations, said vector comprising a deletion of the promoter of the RNA which primes DNA replication.

13. The vector according to claim 12, characterized by a deletion, insertion or mutation which inactivates the *rop* gene product.

14. A method for selecting from DNA populations a desired promoter characterized by the steps of inserting into the vector of claim 12 or 13 DNA fragments from said population and selecting the vectors in which DNA replication occurs.

## Patentansprüche

1. Verbesserter Expressionsvektor, umfassend einen inaktiven oder entfernen Primer-RNA-Promotor, einen anderen Promotor, der funktionsfähig mit der in dem Vektor die Primer-RNA kodierenden DNA-Sequenz verknüpft ist, und mindestens eine Restriktionsschnittstelle, wobei eine ein gewünschtes Polypeptid kodierende DNA-Sequenz in den Vektor eingefügt und darin funktionsfähig mit einer Expressionskontrollsequenz verknüpft werden kann.

2. Expressionsvektor nach Anspruch 1, dadurch gekennzeichnet, daß der andere Promotor der RNA I-Promotor, der lacUV5-Promotor oder ein Derivat davon ist.

3. Expressionsvektor nach Anspruch 1 oder 2, gekennzeichnet durch eine Deletion, Insertion oder Mutation, welche das *rop*-Genprodukt inaktiviert.

4. Expressionsvektor nach einem der Ansprüche 1 bis 3, dadurch gekennzeichnet, daß die Expressionskontrollsequenz gekennzeichnet ist durch den Promotor, der funktionsfähig mit der die Primer-RNA kodierenden DNA-Sequenz verknüpft ist.

5. Expressionsvektor nach Anspruch 4, dadurch gekennzeichnet, daß die Expressionskontrollsequenz auch für Ribosomenbindungsstellen kodierende Sequenzen einschließlich der Shine-Dalgarno-Sequenzen umfaßt.

6. Expressionsvektor nach Anspruch 1 bis 5, dadurch gekennzeichnet, daß er pRN16 (Zugangsnummer DSM 2929), pLA51 (Zugangsnummer DSM 2930) oder pLA512 (wie in der Beschreibung dargestellt) ist.

7. Expressionsvektor nach einem der Ansprüche 1 bis 3, dadurch gekennzeichnet, daß die Expressionskontrollsequenz gekennzeichnet ist durch einen Promotor, der nicht funktionsfähig mit der die Primer-RNA kodierenden DNA-Sequenz verknüpft ist.

8. Expressionsvektor nach einem der Ansprüche 1 bis 7, dadurch gekennzeichnet, daß er auch eine ein eukaryontisches oder prokaryontisches Polypeptid kodierende, in den Vektor an der Restriktionsschnittstelle eingefügte und darin funktionsfähig mit der Expressionskontrollsequenz verknüfte DNA-Sequenz umfaßt.

9. Expressionsvektor nach Anspruch 8, dadurch gekennzeichnet, daß die DNA-Sequenz eine tierische und menschliche Hormone, virale und bakterielle Antigene oder andere eukaryontische und prokaryontische Polypeptide kodierende DNA-Sequenz ist.

10. Expressionvektor nach Anspruch 9, dadurch gekennzeichnet, daß die DNA-Sequenz Polypeptide, nämlich menschliche und tierische Interferone, menschliche und tierische Wachstumshormone, MKSV-Antigene, HBV-Antigene, menschliches Insulin, menschliche Blutfaktoren, Gewebeplasminogenaktivator oder Erythropoietin kodiert.

11. Verfahren zur Herstellung eines Polypeptides, gekennzeichnet durch die Schritte einer Kultivierung eines mit einem Vektor nach einem der Ansprüche 8 bis 10 transformierten Wirtes und Gewinnung der Polypeptide.

12. Verbesserter Vektor zur Vewendung bei der Auswahl von Promotoren aus DNA-Populationen, wobei der Vektor eine Deletion der Primer-RNA für die DNA-Replikation umfaßt.

13. Vektor nach Anspruch 12, gekennzeichnet durch eine Deletion, Insertion oder Mutation, welche das *rop*-Genprodukt inaktiviert.

14. Verfahren zur Auswahl eines gewünschten Promotors aus DNA-Populationen, gekennzeichnet durch die Schritte einer Insertion von DNA-Fragmenten aus der Population in den Vektor nach Anspruch 12 oder 13 und Auswahl der Vektoren, bei denen es zu Replikation kommt.

**Revendications**

1. Vecteur d'expression amélioré caractérisé en ce qu'il comprend un promoteur inactif ou supprimé d'un ARN d'amorçage, un autre promoteur qui est lié opérativement à la séquence d'ADN qui encode l'ARN d'amorçage dans le vecteur, et au moins un site de restriction dans lequel une séquence d'ADN qui encode un polypeptide désiré peut être insérée dans le vecteur et liée opérativement dans celui-ci à une séquence de contrôle d'expression.

2. Vecteur d'expression selon la revendication 1, caractérisé en ce que l'autre promoteur est choisi dans le groupe formé par le promoteur d'ARN I, le promoteur de *lac* UV5 et leurs dérivés.

3. Vecteur d'expression selon l'une quelconque des revendications 1 et 2, caractérisé par une suppression, une insertion ou une mutation qui rend inactif le produit du gêne *rop*.

4. Vecteur d'expression selon l'une quelconque des revendications 1 à 3, caractérisé en ce que la séquence de contrôle d'expression est déterminée par le promoteur qui est lié opérativement à la séquence d'ADN qui encode l'ARN d'amorçage.

5. Vecteur d'expression selon la revendication 4, caractérisé en ce que la séquence de contrôle d'expression comprend également des séquences de codage pour les sites de liaison du ribosome, y compris les séquences Shine/Dalgarno.

6. Vecteur d'expression selon l'une quelconque des revendications 1 à 5, caractérisé en ce qu'il est choisi dans le groupe formé par les p RN 16 (numéro de collection DSM 2929), pla 51 (numéro de collection DSM 2930) et pla 512 (comme décrit dans la description).

7. Vecteur d'expression selon l'une quelconque des revendications 1 à 3, caractérisé en ce que la séquence de contrôle d'expression, est déterminée par un promoteur qui n'est pas relié opérativement à la séquence d'ADN qui encode l'ARN d'amorçage.

8. Vecteur d'expression selon l'une quelconque des revendications 1 à 7, caractérisé en ce qu'il comprend également une séquence d'ADN qui encode un polypeptide encaryotique ou procaryotique inséré dans le vecteur au site de restriction et relié opérativement dans celui-ci à la séquence de contrôle d'expression.

9. Vecteur d'expression selon la revendication 8, caractérisé en ce que la séquence d'ADN est choisi dans le groupe formé des séquences d'ADN qui encodent les hormones humaines et animales, les antigènes viraux et bactériens et les autres polypeptides Eucaryotiques et procaryotiques.

10. Vecteur d'expression selon la revendication 9, caractérisé en ce que la séquence d'ADN encode des polypeptides choisis dans le groupe constitué par les interférons humains et animaux, les hormones de croissance humaines et animales, les antigènes du virus FMD, les antigènes du virus de l'hépatite B, l'insuline humaine, les facteurs sanguins humains, l'activateur du plasminogène tissulaire (TPA) et l'érythropoietine.

11. Procédé de production d'un polypeptide caractérisé en ce qu'il comprend les étapes de culture d'un hôte transformé par un vecteur selon l'une quelconque des revendications 8 à 10, et de récolte de ces polypeptides.

12. Vecteur amélioré destiné à être utilisé dans des promoteurs choisis à partir de populations d'ADN, caractérisé en ce qu'il comporte une suppression du promoteur de l'ARN qui amorce la reproduction de l'ADN.

13. Vecteur selon la revendication 12, caractérisé par une suppression, une insertion ou une mutation qui inactive le produit du gène *rop*.

14. Procédé de sélection à partir des populations d'ADN, d'un promoteur désiré, caractérisé en ce qu'il comporte les étapes d'insertion dans le vecteur selon la revendication 12 ou la revendication 13, de fragments d'ADN provenant de la population et de sélection des vecteurs dans lesquels la reproduction de l'ADN se produit.

# FIG. 1

# FIG.2

# FIG.3

EP 0 179 786 B1

*FIG. 4*

# FIG.5

## FIG. 6

# FIG.7

_Afl II_
_T7 rbs_
_P_
_α2_
_AmpR_
_Afl II_
_552/186 α2_
_227/942 col El_
_pLA 512_
_RNA I_
_Pst I_
_ori_
_pBR 322/col El_

———— pBR322 from 4,359 (_EcoRI_) to 3,102 (_Tha I_)

———— col El from -39 (_Pvu II_) to +942

—·—·— IFN-α2 from 227 to 186 and 552 to 1

═══ phage T7 rbs

++++ lac uv5 promoter
(or PI promoter)
or other

# FIG.8

# FIG.9